(19) Europäisches Patentamt
European Patent Office
Office européen des brevets

(11) Veröffentlichungsnummer: **0 158 932**
**B1**

(12) # EUROPÄISCHE PATENTSCHRIFT

(45) Veröffentlichungstag der Patentschrift: **01.04.87**

(51) Int. Cl.⁴: **C 07 C 121/78,** C 07 C 120/00

(21) Anmeldenummer: **85104068.3**

(22) Anmeldetag: **03.04.85**

(54) **Verfahren zur Herstellung von 3-Cyano-4-aminoacetophenonen.**

(30) Priorität: **18.04.84 DE 3414628**

(43) Veröffentlichungstag der Anmeldung: **23.10.85 Patentblatt 85/43**

(45) Bekanntmachung des Hinweises auf die Patenterteilung: **01.04.87 Patentblatt 87/14**

(84) Benannte Vertragsstaaten: **AT BE CH DE FR GB IT LI LU NL SE**

(56) Entgegenhaltungen:
**DE - A - 2 337 951**
**DE - A - 2 354 961**
**US - A - 4 407 819**

**CHEMICAL ABSTRACTS, Band 75, Nr. 9, 30. August 1971, Columbus, Ohio, USA, L. MÜNCHAUSEN et al. "New photochemical addition reactions of acetylenes II. Synthetic potential of neighbouring group participation in photolytic hydration", Seite 376, Zusammenfassung Nr. 62810w**

(73) Patentinhaber: **Dr. Karl Thomae GmbH, Postfach 1755, D-7950 Biberach (Riss) (DE)**

(72) Erfinder: **Resemann, Wolfgang, Dr. Dipl.-Chem., Rosenstrasse 25, D-7950 Biberach 1 (DE)**
Erfinder: **Fraunberger, Ferdinand, Dr. Dipl.-Chem., Sebastian-Kneipp Strasse 16, D-7950 Biberach 1 (DE)**

**EP 0 158 932 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von 3-Cyano-4-aminoacetophenonen.

Die 3-Cyano-4-aminoacetophenone besitzen die allgemeine Formel

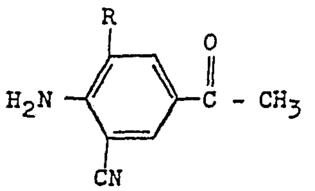
, (I)

in der R ein Wasserstoff- oder Fluoratom darstellt.

Diese Verbindungen sind wichtige Zwischenprodukte zur Herstellung wertvoller Arzneistoffe und Futterzusatzstoffe; als Futterzusatzstoffe bewirken die weiter unten beschriebenen Verbindungen der allgemeinen Formel V in der Tiermast eine verstärkte Futtermittelverwertung, in der Tieraufzucht ein rascheres Wachstum und für die Tierverwertung eine Erhöhung des Verhältnisses von Magerfleisch zu Fett.

Angesichts eines weltweiten Mangels an hochwertiger Eiweissnahrung besteht ein hohes Interesse an derartigen Wirksubstanzen. Zur Bereitstellung ausreichender Mengen dieser Wirksubstanzen ist die Auffindung eines einfach durchführbaren, umweltfreundlichen Herstellverfahrens dringend erwünscht.

Für die Herstellung der Wirksubstanzen nehmen die 3-Cyano-4-aminoacetophenone der allgemeinen Formel I eine wichtige Schlüsselstellung ein. Es galt deshalb, einen einfachen Weg zu ihrer Herstellung zu finden, der nicht nur eine hohe Ausbeute sondern auch eine gute Qualität des Produktes gewährleistet.

Das erfindungsgemässe Verfahren besteht in der Verseifung eines 3-Cyano-4-acetaminoacetophenons der allgemeinen Formel

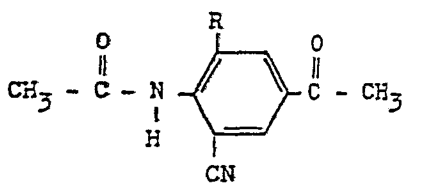
, (II)

in der R wie oben angegeben definiert ist, mittels Alkalihydroxiden oder Alkalimethanolaten in Methanol.

Die Verseifung einer Verbindung der allgemeinen Formel II erfolgt mittels Kaliumhydroxid, Natriumhydroxid oder Kalium- oder Natriummethanolat in Gegenwart von Methanol. Eine bevorzugte Verfahrensweise besteht darin, dass man Kaliumhydroxid in Methanol vorlegt, erwärmt und hierzu, vorzugsweise nach und nach innerhalb einiger Minuten, das 3-Cyano-4-acetaminoacetophenon der allgemeinen Formel II einträgt. Die Verseifung wird durch Erhitzen bis zur Rückflusstemperatur des Reaktionsgemisches zuende geführt. Dampft man das Reaktionsgemisch zur Trockene ein, nimmt den Rückstand in Wasser auf und versetzt die Lösung mit einer Mineralsäure, vorzugsweise mit konzentrierter Salzsäure, so fällt das 3-Cyano-4-aminoacetophenon der allgemeinen Formel I als ein kristalliner Niederschlag aus. Der Niederschlag wird noch nachgewaschen und getrocknet. Das entsprechende 3-Cyano-4-aminoacetophenon der allgemeinen Formel I fällt dabei in reiner Form an. Im allgemeinen erzielt man hierbei Ausbeuten zwischen 80 und 90% der Theorie, bezogen auf die eingesetzte Verbindung der allgemeinen Formel II; ist R ein Fluoratom, so liegen die Ausbeuten etwas tiefer.

Die Verseifung von Verbindungen der allgemeinen Formel II zu den Verbindungen der allgemeinen Formel I unter den obengenannten Bedingungen war nicht vorhersehbar. Die Entacylierung von Verbindungen der allgemeinen Formel II ist bei Anwendung der üblichen Verseifungsmethoden weder in sauren noch in alkalisch wässerigen Systemen in der gewünschten Weise durchführbar. Wie auch aus der Literatur bekannt (vgl. A. Graham, Synthetic Communications 10 (3), 241-243 (1980)) führt die Nachbarstellung einer reaktiven Cyanogruppe neben einer Acylaminogruppe mit starken Säuren zu einer Ringschlussreaktion gemäss nachstehendem Reaktionsschema:

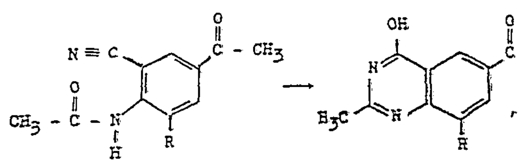
, (III)

oder mit verdünnten Säuren zu dem entsprechenden 2-Acylaminobenzamid. Im übrigen wurde beobachtet, dass in wässerigem alkalischen Medium ebenfalls eine Verseifung der Cyanogruppe zur Säureamidgruppe erfolgt.

Diese Methode dient zur präparativen Gewinnung kondensierter Pyrimidine.

Durch das erfindungsgemässe Verfahren gelingt es zum ersten Mal, die Entacylierung des Acetylrestes unter Erhalt der Cyanogruppe durchzuführen; diese Entacylierung ist nur möglich, wenn Alkalihydroxyde in Methanol oder Alkalimethanolate in Methanol zur Einwirkung gebracht werden.

Die Ausgangsverbindungen der allgemeinen Formel II erhält man beispielsweise durch Umsetzung eines 3-Brom-4-acetaminoacetophenons der allgemeinen Formel

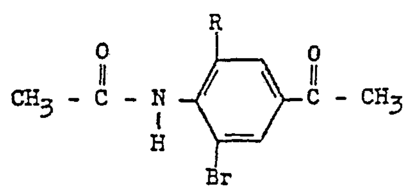
, (VI)

in der R wie eingangs erwähnt definiert ist, mit Kupfer(I)cyanid. Hierzu setzt man das 3-Brom-4-acetaminoacetophenon der allgemeinen Formel IV mit Kupfer(I)cyanid in einem Lösungsmittel, wie Pyridin, Dimethylformamid bei Temperaturen zwischen 100 und 150°C um und reinigt das Produkt z.B. mit Essigsäureethylester (vgl. auch hierzu Houben-Weyl Band 8, Sauerstoffverbindungen III, S 303).

Die Ausgangsverbindungen der allgemeinen Formel IV erhält man beispielsweise aus dem entsprechenden 4-Acetaminoacetophenon (Herstellung siehe Beilstein E II, *14*, 33) durch Bromierung mittels Brom in Gegenwart von Eisessig/Wasser (vgl. L.C. Raiford, H.L. Davis, J. Am. Chem. Soc. *50*, 158 (1928) und Beilstein E II, *14*, 33).

Die Verbindungen der allgemeinen Formel I stellen wichtige Zwischenprodukte bei der Herstellung von Verbindungen der allgemeinen Formel

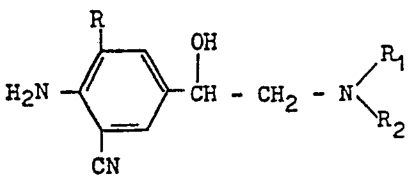

, (V)

dar, worin R wie oben definiert ist, $R_1$ Wasserstoff oder niederes Alkyl und $R_2$ niederes Alkyl bedeuten. Die Verbindungen der allgemeinen Formel V dienen beispielsweise als Futterzusatzstoffe und bewirken ein verstärktes Wachstum bei Schlachtvieh und Geflügel, desweiteren eine verstärkte Futtermittelverwertung; sie erhöhen dabei auch das Verhältnis von Magerfleisch zu Fett (vgl. U.S. Patent 4.407.819). Diese Verbindungen besitzen aber auch therapeutisch verwertbare Eigenschaften, beispielsweise eine Wirkung auf die β-Rezeptoren, insbesondere eine $\beta_2$-mimetische Wirkung. Eine besonders wertvolle Verbindung der allgemeinen Formel V besitzt für R und $R_1$ ein Wasserstoffatom, für $R_2$ die Isopropylgruppe (F. 165-167°C).

Die Verbindungen der allgemeinen Formel V lassen sich in einfacher Weise aus dem 3-Cyano-4-aminoacetophenon durch die folgenden Schritte herstellen:

a) Bromierung in den üblichen Lösungsmitteln, wie Eisessig, Tetrahydrofuran, mit elementaren Brom oder Kupfer(II)bromid,

b) Umsetzung des gebildeten ω-Brom-3-cyano-4-aminoacetophenons mit Aminen der allgemeinen Formel $HNR_1R_2$, in der $R_1$ und $R_2$ wie oben definiert sind, zu den entsprechenden Aminoketonen bei Temperaturen bis 20°C, und

c) Reduktion des Aminoketons zum Aminoalkohol der allgemeinen Formel V mit Hilfe von Natriumboranat in Wasser.

Die folgenden Beispiele sollen das Wesen der Erfindung näher erläutern:

A. Beispiele zur Herstellung der Endprodukte

*Beispiel 1:*

*3-Cyano-4-aminoacetophenon*

Es werden 25,2 g (0,45 Mol) Kaliumhydroxid und 300 ml Methanol in einem Reaktionsgefäss vorgelegt, der Inhalt des Gefässes wird auf 40°C erwärmt. Innerhalb von 2 bis 4 Minuten werden 30 g (0,15 Mol) 3-Cyano-4-acetaminoacetophenon eingetragen, das Reaktionsgemisch wird 20 Minuten bei Rückflusstemperatur erhitzt. Anschliessend wird zur Trockene eingedampft und der Rückstand mit 300 ml Wasser versetzt; man gibt hierzu 40 ml konzentrierte Salzsäure, wobei das gebildete 3-Cyano-4-aminoacetophenon kristallin ausfällt. Man kühlt auf 10°C ab und rührt noch 1 Stunde nach. Nach Absaugen und Nachwaschen des Filterrückstandes mit Wasser und Trocknen desselben bei 60°C erhält man 21 g beigefarbenes 3-Cyano-4-aminoacetophenon, entsprechend einer Ausbeute von 87,5% der Theorie. Schmelzpunkt: 161,5°C.

Bei Verwendung von Natriumhydroxid und Methanol in den gleichen Molverhältnissen und unter den gleichen Umsetzungsbedingungen entsteht das 3-cyano-4-aminoacetophenon in einer Ausbeute von 80% der Theorie.

*Beispiel 2:*

*3-Cyano-4-aminoacetophenon*

Es werden 3,8 kg (59,6 Mol) Kaliumhydroxid und 45,6 l Methanol in einem Reaktionsgefäss vorgelegt, der Inhalt des Gefässes wird auf 40°C erwärmt. Innerhalb von 5 bis 10 Minuten werden 4,58 kg (22,6 Mol) 3-Cyano-4-acetaminoacetophenon eingetragen; man erhitzt auf Rückflusstemperatur für 25 Minuten, entfernt das Lösungsmittel und gibt zu dem trockenen Rückstand 60 l Wasser und hernach 6 l konzentrierte Salzsäure. Die weitere Aufarbeitung erfolgt wie im Beispiel 1 angegeben.

Ausbeute: 3,0 kg 3-Cyano-4-aminoacetophenon entsprechend 82,8% der Theorie.

Schmelzpunkt: 161,5°C.

Mit Kaliummethylat wurde eine Ausbeute von 76% der Theorie erzielt.

*Beispiel 3:*

*3-Cyano-4-amino-5-fluoracetophenon*

In der gleichen Weise, wie im Beispiel 1 beschrieben, wurde 3-Cyano-4-acetamino-5-fluoracetophenon mit Kaliumhydroxid bei 45 bis 50°C und 24-stündiger Reaktionszeit zu 3-Cyano-4-amino-5-fluoracetophenon verseift.

Ausbeute: 55% der Theorie; mit Natriumhydroxid 48% der Theorie.

B. Beispiel zur Herstellung der Ausgangsverbindung 3-Cyano-4-acetaminoacetophenon.

*Beispiel 4:*

In Anlehnung an die in Houben-Weyl, Band 8, Sauerstoffverbindungen III, Seite 303, beschriebene Rosenmund-V.Braun'sche Reaktion werden 70 g (0,273 Mol) 3-Brom-4-acetaminoacetophenon (hergestellt nach der Methode von L.C. Raiford, H.L. Davis, J. Am. Chem. Soc. *50*, 158 (1928)) und 24,5 g (0,273 Mol) Kupfer(I)cyanid in 150 ml Dimethylformamid im Ölbad während 2,5 Stunden auf 130°C erhitzt. Dabei fällt Kupfer(I)bromid aus, das am Ende der Reaktion bei 95°C über eine Filternutsche abgesaugt und mit 20 ml Dimethylformamid nachgewaschen wird. Das warme Filtrat wird in 300 ml Wasser gegossen, wobei das 3-Cyano-4-acetaminoacetophenon und das restliche Kupfer(I)bromid ausfällt. Die Suspension wird 2 Stunden nachgerührt, abgesaugt und der Filterrückstand mit Wasser nachgewaschen. Das bei 60°C getrocknete Rohprodukt

wird zur Entfernung des Kupfersalzes zunächst mit 700 ml Essigsäureethylester und erneut mit 300 ml Essigsäureethylester ausgekocht; die vereinigten Extrakte werden auf 200 ml Gesamtvolumen eingeengt. Zur vollständigen Ausfällung gibt man noch 200 ml Petroläther (60/70°C) zur warmen Essigester-Suspension. Nach dem Abkühlen und Absaugen wird der Filterrückstand bis zur Gewichtskonstanz bei 60°C getrocknet.

Ausbeute: 36 g 3-Cyano-4-acetaminoacetophenon entsprechend 65,2% der Theorie bezogen auf das eingesetzte 3-Brom-4-acetaminoacetophenon.

Schmelzpunkt: 170°C.

## Patentansprüche

1. Verfahren zur Herstellung von 3-Cyano-4-aminoacetophenonen der allgemeinen Formel

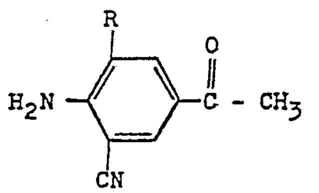

, (I)

in der R ein Wasserstoff- oder Fluoratom darstellt, dadurch gekennzeichnet, dass ein 3-Cyano-4-acetaminoacetophenon der allgemeinen Formel

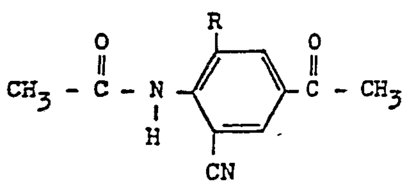

, (II)

in der R wie oben definiert ist, mit Kaliumhydroxid, Natriumhydroxid, Kalium- oder Natriummethanolat in Gegenwart von Methanol als Lösungsmittel bei Temperaturen bis zum Siedepunkt des Reaktionsgemisches verseift und die Verbindung der allgemeinen Formel I aus dem Reaktionsgemisch isoliert wird.

2. Verfahren gemäss Anspruch 1, dadurch gekennzeichnet, dass zur Verseifung Kaliumhydroxid in Methanol verwendet wird.

3. Verfahren gemäss Anspruch 1 und 2, dadurch gekennzeichnet, dass die Verbindung der allgemeinen Formel II in eine bei 40°C gehaltene Kaliumhydroxid-Methanollösung eingetragen, das Reaktionsgemisch anschliessend auf Temperaturen zwischen 45°C und der Rückflusstemperatur erwärmt, nach erfolgter Verseifung das Lösungsmittel Methanol entfernt, der Rückstand in Wasser gelöst und das Endprodukt durch ansäuern ausgefällt wird.

## Claims

1. Process for preparing 3-cyano-4-aminoacetophenones of general formula

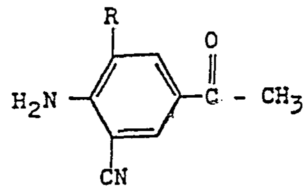

(I)

wherein R represents a hydrogen or fluorine atom, characterised in that a 3-cyano-4-acetaminoacetophenone of general formula

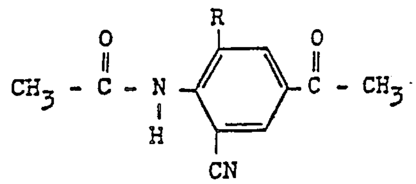

(II)

wherein R is defined as hereinbefore, is saponified with potassium hydroxide, sodium hydroxide, potassium or sodium methoxide in the presence of methanol as solvent at temperatures up to the boiling point of the reaction mixture and the compound of general formula I is isolated from the reaction mixture.

2. Process as claimed in claim 1, characterised in that potassium hydroxide in methanol is used for the saponification.

3. Process as claimed in claims 1 and 2, characterised in that the compound of general formula II is added to a potassiumhydroxide/methanol solution maintained at 40°C, the reaction mixture is then heated to temperatures of between 45°C and reflux temperature, after saponification has taken place the solvent methanol is eliminate, the residue is dissolved in water and the end product is precipitated by acidification.

## Revendications

1. Procédé pour la préparation de 3-cyano-4-amino-acétophénones de formule générale

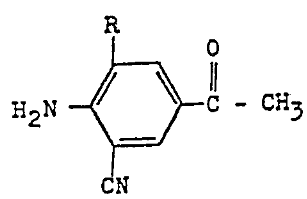

(I)

dans laquelle R représente un atome d'hydrogène ou de fluor, caractérisé en ce qu'on saponifie une 3-cyano-4-acétamino-acétophénone de formule générale

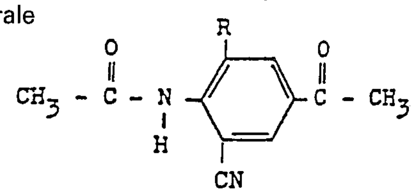

(II)

dans laquelle R défini comme plus haut, au moyen d'hydroxyde de potassium, d'hydroxyde de sodium, de méthanolate de potassium ou de sodium en présence de méthanol comme solvant à des températures allant jusqu'au point d'ébullition du

mélange réactionnel et en ce que l'on isole le composé de formule générale I à partir du mélange réactionnel.

2. Procédé selon la revendication 1, caractérisé en ce que l'on utilise de l'hydroxyde de potassium dans le méthanol pour la saponification.

3. Procédé selon les revendications 1 et 2, caractérisé en ce que le composé de formule générale II est introduit dans une solution d'hydroxyde de potassium dans le méthanol maintenue à 40°C, le mélange réactionnel est ensuite chauffé à des températures entre 45°C et la température de reflux, lorsque la saponification a eu lieu, le solvant méthanol est éliminé, le résidu est dissous dans l'eau et le produit final est précipité par acidification.